# EUROPEAN PATENT APPLICATION

(11) **EP 1 323 821 A1**
(43) Date of publication of application: **02.07.2003**
(21) Application number: 01965613.1
(22) Date of filing: 13.09.2001
(51) Int. Cl.: C12N 5/06, C12N 15/09, A01K 67/00

(54) **PROCESS FOR PRODUCING NORMAL PARENCHYMAL CELLS, TISSUE OR ORGAN BY BIOINCUBATOR**

(30) Priority: 14.09.2000 JP 2000280295
(71) Applicant: Yuki, Atsushi, Saitama-shi, Saitama 330-0023 (JP)
(72) Inventor: Yuki, Atsushi, Saitama-shi, Saitama 330-0023 (JP)
(74) Representative: HOFFMANN EITLE
(86) International application number: JP0107947
(87) International publication number: WO02022789

(57) **Abstract**

Methods of producing cells, tissues and organs as close as possible to homoplasy have been developed to provide nonhuman mammals having normal parenchymal cells of a foreign mammal, a tissue made up of normal cells of a foreign mammal and their secretion products thereof, and/or an organ of a foreign mammal.

SEQUENCE LISTING

<110> Atsushi Yuki
<120> METHOD OF PRODUCING NORMAL PARENCHYMAL CELLS, TISSUES OR
    ORGANS USING LIVE-CULTURE DEVICE
<130> 002223
<160> 11

<210> 1
<211> 320
<212> DNA
<213> rat
<220>
<223> Entire nucleotide sequence of MLCproTK
    Promoter region:1-44
    HSVtk encoding region:168-1811
<400> 1
gatctcggct cttacttcag aagaacggca tggggtgggg gggcttaggt ggcctctgcc   60
tcacctacaa ctgccaaaag tggtcatggg gttattttta accccaggga agaggtattt  120
attgttccac agcaggggcc ggccagcagg atcctgtaga agcgcgtatg gcttcgtacc  180
ccggccatca gcacgcgtct gcgttcgacc aggctgcgcg ttctcgcggc catagcaacc  240
gacgtacggc gttgcgccct cgccggcagc aagaagccac ggaagtccgc ccggagcaga  300
aaatgcccac gctactgcgg gtttatatag acggtcccca cgggatgggg aaaaccacca  360
ccacgcaact gctggtggcc ctgggttcgc gcgacgatat cgtctacgta cccgagccga  420
tgacttactg gcgggtgctg ggggcttccg agacaatcgc gaacatctac accacacaac  480
accgccttga ccagggtgag atatcggccg gggacgcggc ggtggtaatg acaagcgccc  540
agataacaat gggcatgcct tatgccgtga ccgacgccgt tctggctcct catatcgggg  600
gggaggctgg gagctcacat gccccgcccc cggccctcac cctcatcttc gaccgccatc  660
ccatcgccgc cctcctgtgc tacccggccg cgcgatacct tatgggcagc atgacccccc  720
aggccgtgct ggcgttcgtg gccctcatcc cgccgacctt gcccggcaca aacatcgtgt  780
tgggggccct tccggaggac agacacatcg accgcctggc caaacgccag cgccccggcg  840
agcggcttga cctggctatg ctggccgcga ttcgccgcgt ttacgggctg cttgccaata  900
cggtgcggta tctgcagggc ggcgggtcgt ggcgggagga ttggggacag ctttcgggga  960
cggccgtgcc gccccagggt gccgagcccc agagcaacgc gggcccacga ccccatatcg 1020
ggacacgttt atttaccctg tttcgggccc ccgagttgct ggcccccaac ggcgacctgt 1080
acaacgtgtt tgcctgggcc ttggacgtct tggccaaacg cctccgtccc atgcacgtct 1140

ttatcctgga ttacgaccaa tcgcccgccg gctgccggga cgccctgctg caacttacct 1200
ccgggatgat ccagacccac gtcaccaccc caggctccat accgacgatc tgcgacctgg 1260
cgcgcacgtt tgcacgggag atgggggagg ctaactgaaa cacggaagga gacaataccg 1320
gaaggaacct gcgctatgac ggcaataaaa agacagaata aaacgcacgg gtgttgggtc 1380
gtttgttcat aaacgcgggg ttcggtccca gggctggcac tctgtcgata ccccaccgag 1440
accccattgg ggccaatacg cccgcgtttc ttccttttcc ccaccccacc ccccaagttc 1500
gggtgaaggc ccagggctcg cagccaacgt cggggcggca ggcctgccat agccacgggc 1560
cccgtgggtt agggacgggg tcccccatgg ggaatggttt atggttcgtg ggggttatta 1620
tttgggcgtt gcgtggggtc agtccacgac tggactgagc agacagaccc atggtttttg 1680
gatggcctgg gcatggaccg catgtactgg cgcgacacga acaccgggcg tctgtggctg 1740
ccaaacaccc ccgaccccca aaaaccaccg cgcggatttc tggcgccgcc ggacgaacta 1800
aacctgacta c 1811

<210> 2
<211> 171
<212> DNA
<213> rat
<220>
<223> MLC pro
<400> 2
agatctcggc tcttacttca gaagaacggc atggggtggg ggggcttagg tggcctctgc 60
ctcacctaca actgccaaaa gtggtcatgg ggttattttt aaccccaggg aagaggtatt 120
tattgttcca cagcaggggc cggccagcag gctcctgtag aagcgcgtat g 171

<210> 3
<211> 25
<212> DNA
<213> Artificial Sequence
<220>
<223> Primer TK-MET for amplifying cytotoxin gene TK
<400> 3
tttggatcct gtagaagcgc gtatg 25

<210> 4
<211> 37
<212> DNA

<213> Artificial Sequence
<220>
<223> Primer TK-PAA for amplifying cytotoxin gene TK
<400> 4
tttgcggccg cagatctgta gtcaggttta gttcgtc                                37

<210> 5
<211> 20
<212> DNA
<213> Artificial Sequence
<220>
<223> Primer MLCTKF1 for determining nucleotide sequence of
    amplified TK gene
<400> 5
gtctgcgttc gaccaggctg                                                   20

<210> 6
<211> 20
<212> DNA
<213> Artificial Sequence
<220>
<223> Primer MLCTKF2 for determining nucleotide sequence of
    amplified TK gene
<400> 6
aatgacaagc gcccagataa                                                   20

<210> 7
<211> 20
<212> DNA
<213> Artificial Sequence
<220>
<223> Primer MLCTKF3 for determining nucleotide sequence of
    amplified TK gene
<400> 7
gcgagcggct tgacctggct                                                   20

<210> 8
<211> 20
<212> DNA
<213> Artificial Sequence
<220>
<223> Primer MLCTKF3 for determining nucleotide sequence of
    amplified TK gene
<400> 8
tcctggatta cgaccaatcg                                                    20

<210> 9
<211> 20
<212> DNA
<213> Artificial Sequence
<220>
<223> Primer MLCTKF4 for determining nucleotide sequence of
    amplified TK gene
<400> 9
tgaaggccca gggctcgcag                                                    20

<210> 10
<211> 20
<212> DNA
<213> Artificial Sequence
<220>
<223> Primer MLCTKR1 for determining nucleotide sequence of
    amplified TK gene
<400> 10
acttccgtgg cttcttgctg                                                    20

<210> 11
<211> 1683
<212> DNA
<213> Herpes simplex virus
<220>
<223> thymidine kinase

<400> 11
tttggatcct gtagaagcgc gtatggcttc gtaccccggc catcagcacg cgtctgcgtt 60
cgaccaggct gcgcgttctc gcggccatag caaccgacgt acggcgttgc gccctcgccg 120
gcagcaagaa gccacggaag tccgcccgga gcagaaaatg cccacgctac tgcgggttta 180
tatagacggt ccccacggga tggggaaaac caccaccacg caactgctgg tggccctggg 240
ttcgcgcgac gatatcgtct acgtacccga gccgatgact tactggcggg tgctgggggc 300
ttccgagaca atcgcgaaca tctacaccac acaacaccgc cttgaccagg gtgagatatc 360
ggccggggac gcggcggtgg taatgacaag cgcccagata acaatgggca tgccttatgc 420
cgtgaccgac gccgttctgg ctcctcatat cgggggggag gctgggagct cacatgcccc 480
gcccccggcc ctcaccctca tcttcgaccg ccatcccatc gccgccctcc tgtgctaccc 540
ggccgcgcga taccttatgg gcagcatgac cccccaggcc gtgctggcgt tcgtggccct 600
catcccgccg accttgcccg gcacaaacat cgtgttgggg gcccttccgg aggacagaca 660
catcgaccgc ctggccaaac gccagcgccc cggcgagcgg cttgacctgg ctatgctggc 720
cgcgattcgc cgcgtttacg ggctgcttgc caatacggtg cggtatctgc agggcggcgg 780
gtcgtggcgg gaggattggg gacagctttc ggggacggcc gtgccgcccc agggtgccga 840
gccccagagc aacgcgggcc cacgacccca tatcggggac acgttattta ccctgtttcg 900
ggcccccgag ttgctggccc ccaacggcga cctgtacaac gtgtttgcct gggccttgga 960
cgtcttggcc aaacgcctcc gtcccatgca cgtctttatc ctggattacg accaatcgcc 1020
cgccggctgc cgggacgccc tgctgcaact tacctccggg atgatccaga cccacgtcac 1080
caccccaggc tccataccga cgatctgcga cctggcgcgc acgtttgcac gggagatggg 1140
ggaggctaac tgaaacacgg aaggagacaa taccggaagg aacctgcgct atgacggcaa 1200
taaaaagaca gaataaaacg cacgggtgtt gggtcgtttg ttcataaacg cggggttcgg 1260
tcccagggct ggcactctgt cgatacccca ccgagacccc attggggcca atacgcccgc 1320
gtttcttcct tttccccacc ccacccccca agttcgggtg aaggcccagg gctcgcagcc 1380
aacgtcgggg cggcaggcct gccatagcca cgggccccgt gggttaggga cggggtcccc 1440
catggggaat ggtttatggt tcgtgggggt tattatttgg gcgttgcgtg gggtcagtcc 1500
acgactggac tgagcagaca gacccatggt ttttggatgg cctgggcatg gaccgcatgt 1560
actggcgcga cacgaacacc gggcgtctgt ggctgccaaa cacccccgac ccccaaaaac 1620
caccgcgcgg atttctggcg ccgccggacg aactaaacct gactactcta gacgccggcg 1680
ttt 1683

## Description

### FIELD OF THE INVENTION

This invention relates to a nonhuman mammal having cells of a mammal such as a human, a tissue or an organ made up of cells of a mammal such as a human and secretion products of the cells. The invention also relates to a method of constructing a live-culture device having functions in part of a nonhuman mammal, and a method of constructing cells or a tissue or organ of a mammal such as a human using the live-culture device.

This live-culture device is useful for producing any kind of cells, tissues and organs having genes with cell-specific expression, in particular, cells, tissues and organs to be transplanted into humans.

### PRIOR ART

In recent years, public attentions have been paid as epoch-making techniques to: gene therapy for supplementing defected genes; regeneration therapy wherein cells are injected from outside into the site of an injured organ to promote recovery of the injured site; and organ transplantation for exchanging a tissue or an organ having been so seriously injured to make any recovery impossible. Normal human cells, tissues and organs which are required in all of these techniques in common are supplied from the human body. However, it is difficult to obtain the most adequate material in a required amount on demand. Thus, the absolute shortage of these materials is not only a fatal problem but also disturbs advances in these techniques.

Concerning nonhuman mammals, it has been also expected to develop novel techniques, such as for producing less expensive vaccines for infectious diseases and carcinogenesis caused by infection etc., or for adding commercially desired new characteristics to adult animals by transplantation of transgenic cells and so on. However, no established technique is available at present for supplying normal cells required in these expected techniques.

It is relatively easy to obtain nonhuman animal cells, tissues and organs. Therefore, the urgent problem of the shortage of these materials will be solved by establishing a technique of the so-called xenotransplant, i.e., transplantation of cells, tissues or organs of nonhuman animals (in particular, industrial animals) into human patients. In xenotransplant, there arises an urgent problem of super-acute rejection which is an immune reaction of a recipient induced by a foreign antigen of a donor animal. However, this super-acute rejection associating with the transplantation of cells, a tissue or organ has been relieved to some extent by constructing a transgenic pig having a gene encoding a factor inhibiting the first step of this reaction. For example, Immutran in UK reported that a porcine heart having DAF gene introduced therein survived in a cynomolgus monkey for 63 days, Alexion reported that a transgenic porcine heart survived in a baboon for 48 hours, and Nextran reported that a porcine heart having DAF and HRF20 genes introduced therein survived in a baboon for 69 hours.

Pig is excellent in productivity (reproductivity and quick maturation) and can be fed under relatively easily controlled conditions (aseptic rearing, etc.). Moreover, sufficient knowledge in pathogens of pig is available. In addition to these favorable characteristics, porcine organs are similar to human ones in size, structure and biochemical properties. Due to these factors, there have been known many methods of replacing human cells, tissues or organs by porcine ones while controlling rejection, in addition to the methods with the use of transgenic pigs as described above. Such methods include, for example, inactivation of a porcine cardiomyocyte surface antigen by treating with an antibody *in vitro*, inhibition of the biosynthesis of cell surface layer antigens by genetic manipulation (universal donor cells), removed of cells from a construct such as porcine cardiac valve by chemical treatments, and so on. These methods of introducing genes use transgenic pigs of novel varietics which inherently have an organs capable of constantly producing a substance capable of inhibiting immunological rejection. Thus, they are useful for the mass production of cells, tissues and organs for xenotransplant.

On the other hand, the situation in the field of production of normal human cells has greatly changed due to the invention of human embryonic stem cells. There have been reported a system for differentiating mouse myocytes, nerve cells, blood cells, etc. *in vitro* by chemically treating mouse embryonic stem cells and a system of constructing embryoids. Furthermore, a patent has been granted to a method of producing mouse cardiomyocytes *in vitro* by genetically manipulating mouse embryonic stem cells.

However, the property of embryo stem cells largely depends on various genetic factors which still remain unknown such as species, strain, sex and so on of the source animal. Accordingly, techniques developed using typical mouse embryo stem cells are not always applicable as such to human embryo stem cells obtained hitherto. Although extensive studies have been made over a long time, no such embryo stem cell as defined in mouse (namely, a totipotent cell line capable of differentiating into reproductive cells) has been established in animal species other than mouse (for example, cattle and pig).

Although Rhesus monkey and human embryonic stem cells, have been reported, the potency of these cells are based on standards different from the totipotent cells as defined in mouse. That is to say, it is still unknown whether or not these cells can be differentiated into any human somatic cells similar to human egg cells. Therefore, the development of human embryonic stem cells per se at the present stage cannot directly contribute to the production of human tissues and organs having three-dimensional structures with differentiated. Although attempts have been made to produce artificial organs by adding cells of the organs to an artificial construct prepared by using synthetic chemicals or natural polymers, it is impossible to alter the structure or composition of such artificial construct. Thus, only a part of the cell population constituting organs can be obtained so far. Therefore, only some parts of natural tissues or organs can be substituted thereby.

### DISCLOSURE OF THE INVENTION

The present invention aims at providing techniques of producing cells or a tissue or organ (a live-culture device) as close as possible to allotransplant from a viewpoint different from the conventional ones.

More specifically, the present invention aims at providing a method of producing an organ as close as possible to a human organ in a non-human mammal without resort to any human totipotent embryonic stem cells and a live-culture device to be used in this method. Using the live-culture device according to the present invention, it is possible to produce cells or a tissue or organ, etc., which cannot be prepared by using a fixed culture device by the somatic cell nuclear transplantation or with the use of human embryonic stem cells or the like.

### BRIEF DESCRIPTION OF THE DRAWINGS

Fig. 1 shows the nucleotide sequence of rat MLC2v promoter region (250 bp).
Fig. 2 shows the nucleotide sequence of MLCpro.
Fig. 3 shows the entire nucleotide sequence of HSVTK.
Fig. 4 shows a detailed process of the construction of pQBIMLCTK.
Fig. 5 shows a chimeric embryoid body constructed in the presence of ganciclovir.

### DETAILED DESCRIPTION

The present invention has been made based on the idea of enabling the production of cells, a tissue and an organ as close as possible to allotransplant without resort to the human body by constructing a nonhuman mammal having human cells or a tissue or organ made up of human-origin cells and secretion thereof, different from the conventional concept of producing cells, tissues or organs for transplantation into a recipient by the method of introducing a cloned gene into a nonhuman mammal.

It is adequate for the present invention to employ a nonhuman mammal having a function relating to the production of the target cells or a tissue or organ among various functions thereof. That is to say, the present invention uses such a nonhuman mammal as a live-culture device from a novel viewpoint which differs from the conventional concepts of livestock or wild animals which have been bred in order to produce milk or meat. Different from pig under breeding for meat production, for example, it is unnecessary in the present invention to consider meat qualities or yield. Also, the agility of wild boar is not required. Furthermore, it also becomes unnecessary to consider various abilities needed in surviving in farm breeding conditions (for example, disease-tolerance, weather-tolerance, learning ability, eating ability, digesting ability, etc.) or abilities which are needed in surviving in nature and had to be considered in breeding facilities. On the other hand, in the present invention, greater importance is attached to the following characteristics, i.e., being free from any infectious pathogens harmful to human body which will cause troubles when transplanted into the human body, being free from cells cancerating in the human body, the target cells, tissue or organ having similar morphological and biochemical properties as those of humans, being capable of normally proliferating and growing in a closed breeding chamber to prevent infection, and having excellent biological properties concerning gene manipulations and embryo manipulations (for example, being capable of providing a large number of ova, being highly reproductive, both of males and females quickly growing to the sexual maturation stage, easily providing sample materials for analyzing the phenotype characteristics of the next generation, and allowing breeding on a large scale).

The method according to the present invention also differs from the method of producing a semi-artificial organ by growing totipotent cells, pluripotent cells or somatic cells capable of proliferating *in vitro* on a three-dimensional artificial construct. That is to say, the live culture device according to the present invention does not have a fixed structure so that it undergoes morphological and biochemical changes in the course of proliferation and differentiation of the cells and in the course of the development of the intended tissue or organ. Thus, the present invention provides a culture device based on a novel concept developed from but partly includes the conventional idea of cell culture devices.

There have been reported some practical methods for culturing cells, which have been processed *in vitro*, in a live body, typified by a method of culturing cells injected into the abdominal fluid of a hamster and a method of injecting blood cells through a blood vessel. In these methods, the injected cells would not form any construct such as a tissue or an organ and it is unnecessary to provide any construct for obtaining the target cells. Thus, they are different from the shape-flexible live-culture device according to the present invention.

As a method similar to the above examples, it was reported that sperm precursor cells were transplanted into a sperm-producing organ (i.e., testis) of a foreign animal to yield matured sperms. However, the spermatic fluid produced by this method is not a cell mass (a tissue or an organ) constructed in accordance with same structural rule but regarded as a suspension of individual cells. Therefore, this method is also different from the method according to the present invention.

The method according to the present invention of producing normal parenchymal cells, a tissue or an organ (hereinafter referred to as "organ or the like") in a mammal comprises the following steps:
(1) preparing cells which are obtained from a mammal of the same species as that of the target organ or the like to be produced and capable of differentiating at least into the target organ or the like (hereinafter referred to as "cells for organ"), for example, pluripotent cells originating in human embryonic stem cells;
(2) preparing cells which are obtained from a nonhuman mammal of a species different from that of the target organ or the like to be produced and have pluripotency or totipotency (hereinafter referred to as "cells for culture device"), for example, porcine embryo cells;
(3) introducing a DNA fragment containing a promoter region functioning specifically in the target organ or the like to be produced and a gene encoding an activated cytotoxin or an enzyme activating a cytotoxin precursor which is ligated under the control of said promoter region into the genome of the cells for culture device as described in (2);
(4) culturing the cells for organ as described in (1) together with the cells for culture device as described in (3) to form a chimeric cell mass;
(5) culturing the chimeric cell mass under conditions adequate for the development thereof to provide a live-culture device made of a mammal mainly having the characters originating in the cells for culture device, its embryo or its embryoid body, and specifically expressing the activated cytotoxin or the cytotoxin precursor activating enzyme in the cells (for example, cardiomyocytes) originating in the cells for culture device of the target organ or the like formed in the course of the development of the live-culture device and, in the case of expressing the cytotoxin precursor activating enzyme, adding the cytotoxin precursor; and
(6) allowing the live-culture device to produce the target organ or the like which is made up of the cells originating in the cells for organ and substantially free from cells originating in the cells for culture device.

### Organs or the like which can be produced

Examples of the mammalian normal parenchymal cells, a tissue or an organ (organ or the like) which can be produced by the method according to the present invention include normal parenchymal cells such as cardiomyocytes, pancreatic β-cells, hepatic parenchymal cells, nerve cells, gliacytes and pituitary β-cells, tissues such as Langerhans islet of pancreas, mammary epithelium, vascular endothelium, pituitary anterior lobe and dienthephalon hypothalamus, and organs such as heart, kidney, liver and mamma. However, the present invention is not restricted to the examples cited above but applicable to any cells, tissues and organs having a promoter region functioning specifically in the target cells, tissue or organ and a promoter region wherein the expression of a cytotoxin or an enzyme activating a cytotoxin precursor does not inhibit the production of the target cells, tissue or organ.

### Cells for culture device

It is preferable that the cells for culture device are totipotent or pluripotent cells originating in a mammal having an organ or the like closely similar in size, functions and properties to the organ or the like to be produced of the mammal.

In the case where a complete mammalian individual is to be used as the live-culture device, totipotent cells are employed as the cells for culture device. As the totipotent cells, mammalian embryo cells up to the early stage of the cardiomyocyte formation can be usually used. However, because of technical constraints relating to the transplantation of foreign cells, embryonic cells up to the blastocyst stage are to be employed. In case of aiming at producing a heart substantially made up of human cells, for example, it is appropriate to use pig totipotent cells, in particular, embryonic cells up to the early stage of the cardiomyocyte formation as the cells for culture device. Because of technical constraints relating to the transplantation of foreign cells, embryonic cells up to the blastocyst stage is to be employed in practice. It is also possible to use embryonic stem cells capable of differentiating into any cells as a substitute for the fertilized egg cells. In the strict meaning, embryonic stem cells are defined as cells capable of differentiating any cells constituting an individual including reproductive cells. In mouse, embryonic stem cells, which can be expanded in the undifferentiated state *in vitro* and subsequently differentiated and grown into a complete individual, have already been obtained (Evans, MJ, Kaufman MH: Nature 292: 154-156 (1981); Martin G: Proc. Natl. Acad. Sci. USA 78: 7634-7638 (1981)). When such embryonic stem cells of a mammalian species are obtained as the cells for culture device, use may be made of these cells.

It is not always necessary that the live-culture device according to the present invention is a complete mammalian individual. In animals other than mouse (e.g., cattle, pig, etc.), it seems difficult to obtain embryo-origin cells capable of differentiating into reproductive cells such as sperm and ovum, i.e., embryonic stem cells in the strict meaning. However, there have been known embryo-origin pluripotent cells capable of differentiating into several types of somatic cells which are usable in tests. In the present invention, these pluripotent cells can be also employed as cells for culture device. In this case, a chimera formed by the cells for culture device together with the cells for organ does not grow to be a complete individual but forms, for example, am embryoid body (Thomson JA, Kalishman J, Golos TG, Suring M, Harris CP, Becker RA, Hearn JP: Proc. Natl. Acad. Sci. USA 92: 7844-7848 (1995)) and then the embryoid body forms the target tissue etc. made up of cells originating in the cells of the organ or the like.

### Cells for organ

The mammalian species of the organ or the like to be produced according to the present invention is not particularly restricted. That is to say, it is required that cells for organ maintain the capability of differentiating into the target organ or the like. As cells having this capability, totipotent cells such as embryonic stem cells show the highest possibility of differentiating into target cells. From the technical viewpoint, therefore, it is most convenient to use embryonic stem cells. However, cells for organ are sufficient if they are capable of differentiating into target cells, tissue or organs. Thus, use may be also made of pluripotent cells originating in embryo or embryonic stem cells, stem cells for a tissues or organs, or initialized somatic cells.

To achieve one of the objects of the present invention of producing an organ or the like substantially made up exclusively of human-origin cells, it is preferable to use human pluripotent cells as the cells for organs. In this case, it is not necessary that the cells differentiate into reproductive cells. From the viewpoint of bioethics, on the other hand, the differentiation into reproductive cells should be prevented. In the case of human cells, therefore, it is particularly inadequate to use embryonic stem cells defined in the original meaning. Namely, it is desirable to use the target cells or cells destined to differentiate into the target tissue or organ, i.e., stem cells for the target cells, tissue or organ. From this point of view, human embryo stem cells invented by researchers in Wisconsin University and Johns Hopkins University can be mentioned as human pluripotent cells usable in the present invention. It has never been found that these human cells differentiate into either reproductive cells or human individuals (Wisconsin University (prepared from human blastocyst), Thomson JA, Itskovitz-Eldor J, Shapiro SS, Waknitz MA, Swiergiel JJ, Marshal VS, Jones JM: Science 282: 1145-1147 (1998); Johns Hopkins University (prepared from aborted fetus), Shamblott MJ, Axelman J, Wang S, Bugg EM, Littlefield JW, Donovan PJ, Blumenthal PD, Huggins GR, Gearhart JD: Proc. Natl. Acad. Sci. USA: 95: 13725-13731 (1988)).

The important point of the somatic nuclear transplantation method, which attracted public attention because of the construction of the Dolly clone sheep, resides in the invention of an initialization technique which comprises treating differentiated somatic cells *in vitro* and then injecting the cells into a denucleated ovum to thereby recover the nuclear characteristics of undifferentiated cells. Thus, the previously believed theory was overturned by showing that differentiation of cells is reversible and a nucleus once having differentiated can be restored into the undifferentiated state. Although cytoplasmic factors required for the initialization have not been sufficiently clarified yet, it is obvious to a person skilled in the art that the present invention may include a method relying on the reversibility of cells.

Namely, embryonic stem cells are preferable as the cells for organ in the production of organs which are as close as possible to human organs. However, it is also possible to use cells having a high extent of differentiation, or cells capable of differentiating into a tissue other than the target organ, as a substitute for the embryonic stem cells, cells capable of differentiating into target organs or the like. For example, in the case of constructing a live-culture device by co-culturing cells differentiating into a human skeletal muscle as the cells for organ with the cells for culture device in order to produce a heart close to a human heart, the cells for organ are will be initialized to the undifferentiated state and thus the heart formed in the live-culture device contains human heart cells. Such a case is also included in the scope of the present invention.

### Cytotoxin and its gene

The cytotoxin to be used in the present invention may be an activated cytotoxin such as diphtheria toxin. Taking the mass production of the live-culture device and convenience of operations into consideration, however, it is more preferable to use a gene encoding an enzyme activating a cytotoxin precursor which can exert its cytotoxic effect if necessary, i.e., a protein capable of activating a substance harmless to cells, tissues, organs or individuals into a cytotoxin such as HSVtk (herpes symplex virus thymidine kinase) or CD (bacterial cytosine deaminase).

Although HSVtk per se has no toxicity, it activates ganciclovir and thus exerts a cytotoxic effect (Mullen CA et al: Proc. Natl. Acad. Sci. USA 95: 13725-13731 (1998); Moolten FL, Cancer Res. 46: 5276 (1986)).

Although CD per se has little toxicity, it converts 5'-fluorocytosine into toxic 5'-fluorouridine and thus exerts a cytotoxic effect. Combination of a cytotoxin precursor with a gene product activating the same is not restricted to the two examples as cited above but use can be also made of various combinations reported by, for example, Searle et al. (Brit. J. Cancer 53: 377-384 (1986)).

### Promoter

The nucleotide sequence of the promoter etc. regulating the expression of the gene encoding the cytotoxin is appropriately selected depending on the target cells, tissue or organ. In case of aiming at the production of myocardial cells, for example, use is made of a gene specifically expressed in myocardial cells such as rat myosin light chain 2v gene, myosin light chain Iv promoter, β-myosin heavy chain gene promoter, β-myosin, etc. Similarly, there have been found genes which are expressed specifically in respective cells, and examples of them such promoters known today include insulin gene expressed in pancreatic β-cells and serum albumin expressed in hepatic cells. Accordingly, the present invention can be practiced with almost any cells, tissues and organs employing the promoter region of a gene functioning specifically in the target cells in place of the promoter region of a foreign DNA employed, or by using a plural number of recombinant DNAs in combination.

It is most desirable that the promoter region of a gene as described above is a region specifically functioning in cells participating in the formation of target cells, tissue or organ. However, use can be also made an promoter region functioning in other cells, so long as the function and survival of the live-culture device are not disturbed by the expression of a cytotoxin.

The function of the promoter region a gene often varies depending on the position on the chromosome (position-effect). Thus, it is preferable that the function of the above-described promoter region a gene has been confirmed at the individual level by constructing a transgenic animal.

It is also preferable that the sequence of the above-described promoter region has been clearly and sufficiently specified in order to fully exhibit the function in the target cells.

### Gene introduction into cells for culture device

### Formation of chimera with cells for organ

Methods of forming a chimeric embryo involve the mixed culture method, the aggregation method and the injection method in the order of the development phase of the cells for organ. The injection method is classified into a method wherein the cells for organ are injected into a live-culture device at or before the blastocyst stage outside the maternal body, and another method wherein the cells for organs are injected into a live-culture device after implantation in the uterine. In the mixed culture method which will be illustrated in Examples hereinafter, the cells for culture device forming embryoid bodies and the undifferentiated cells for organ are cultured in a suspended state at a mixing ratio having been predetermined by testing, and then chimeric masses formed due to the adhesiveness of the cells at the stage are developed and differentiated into chimeric embryoid bodies. Although the strong adhesiveness of the cells until the morula stage is employed in the aggregation method too, foreign embryos at or before the morula stage are lightly pressed under a stereomicroscope in this case, different from the mixed culture method. Since the embryos in this stage are covered by a clear zone, the latter is removed by treating with an acid or a protease and then the thus exposed embryos are adhered. The embryos thus adhered are cultured *in vitro* until the early blastocyst stage and those forming a single blastocyst are selected as chimeric embryos to be transplanted into the uterine of a female under pseudo pregnancy. In accordance with the injection method, the cells for organ in the development stage corresponding to an inner cell mass (for example, an inner cell mass of an embryo in the blastocyst stage or embryo stem cells of a different animal species) are mechanically injected into the blastocoel of an embryo in the blastocyst stage, by operating a micromanipulator under a differential interference microscope. Since the chimera formation ratio varies depending on the number of injected cells, the cells are injected in a pre-determined number depending on the species of the both animals employed or the cell strains. After the completion of the injection, the blastocyst is transplanted into the uterine of a female under pseudo pregnancy. The method of injecting the cells for organs into the embryo after the implantation is appropriately selected depending on the animal species of the embryo employed as the live-culture device, the development stage, the target organ and other factors. In general, the cells for organs are directly injected into the embryo exposed by abdominal section.

### Construction of live-culture device

To construct the live-culture device, a recombinant DNA is first constructed by combining a promoter region functioning specifically in the target cell, tissue or organ and a gene of an enzyme activating a cytotoxin precursor. Next, this recombinant DNA is introduced into the genome of the cells for the culture device, and cells wherein the transferred gene normally functions are screened. In the first stage of the screening, cells having the DNA integrated therein are selected with the use of a screening marker ligated on the same DNA molecule as the recombinant DNA. As the screening marker, use is made of GFP (green fluorescent protein) gene encoding a fluorescent protein, G418 registant gene, etc. It is also possible to use a combination of such markers. In the second stage, it is desirable to confirm the death of the target cells during the differentiation stage by adding the cytotoxin precursor.

### Selective removal of cells by activating cytotoxin

When the cytotoxin precursor is added to the live-culture device constructed by the above-described method, those cells in which the activating enzyme is expressed in a sufficient amount will die out. In the case where the live-culture device is an embryoid body, the cytotoxin precursor is added to the medium. The timing of the addition is determined depending on the target cells, tissue or organ. In the case of producing an organ, particular attention should be paid so that the cells of the animal species employed as the live-culture device are removed stepwise depending on the repair ability of the cells for organ to thereby prevent the organ from morphological abnormality.

### Construction of chimera of distantly related species

Another important aspect of the present invention resides in the fact that a chimera have been constructed between distantly related species having different developmental biological properties.

Chimeric animals, which are individuals having cells of different individuals mixed together, appear either non-artificially or they can be artificially constructed. Construction of a chimeric animal between animals of the same species (an allogeneic chimera) has been established as an embryological technique and contributes to the clarification of cell differentiation process. Also, there has been reported the construction of chimeric animals between species having relatively similar developmental biological properties such as goat and sheep (chimera between closely related species). Chimeric animals having cells and properties of both of goat and sheep normally grew after birth.

In recent years, human cells are frequently transferred into nonhuman animals. Injection of human cells into nude mice having hereditarily deteriorated immunological functions has been established as an experimental procedure. A goat fetus having human blood stem cells injected thereinto normally grew after birth and continued produced normal human blood cells into its blood over 2 years. However, blood cells are not cells which form a tissue. Moreover, even if tissue-forming cells are into a mouse, the fixation position of the injected cells and the structure of the resulting cell mass will differ from the inherent ones.

Since in a fetus before birth the immune mechanism has not been completely established yet, it recognizes a foreign antigen in contact at this stage as a self-antigen throughout its lifetime (immune tolerance). It is therefore preferable to inject foreign cells in the fetal stage in which immune tolerance can be established, in particular, before the development of the target tissue or organ. However, contact between cells is closer at earlier development stage of an individual and thus there still remains a possibility that the fetus is affected by the difference in properties between animal cells of different species.

It has not been sufficiently clarified as to at which stage the animal species-specificity is determined during the course of the development and differentiation of a fertilized egg. It is also unknown whether a specific gene determines the species-specificity of an animal or slight differences among individual genes are accumulated and induce properties characteristic to an animal species.

In spite of the apparent differences among animal species, the fundamental functions and structures of cells, tissues and organs constituting mammals and biological substances (nucleic acids, proteins, sugars, etc.) involved in life activities are common throughout mammals. Further, the development course of forming an individual from a fertilized egg is fundamentally the same. That is to say, mammals have a common development process which comprises fertilization between sperm and ovum, division of the fertilized egg and development of a morula, formation of a blastocyst comprising a differentiated cytrophoblost and an inner cell mass, implantation in the uterine, development of germ layers (ectoderm, endoderm and mesoderm) serving as the parent body of all of the organs appearing in the course of the individual development, determination of the differentiation ability of cells toward a specific direction, differentiation of the germ layers, and the subsequent differentiation and development of various tissues and organs originating in respective germ layers and the birth of the infant.

Although the pregnancy period and infant size widely vary form species to species, the development speed until the blastocyst stage does not so widely vary. Namely, cell divisions proceeds at relatively similar speeds in this stage. In the early stage of development, it cannot be always said that a fetus of a large-sized animal is bigger than a fetus of a small-sized animal. This is because a smaller-sized animal has a shorter pregnancy period and thus its development proceeds more quickly. In the fourth week of pregnancy, for example, a fetus of a medium-sized animal (for example, pig) is 1.5 to 3 times as big as a fetus of a large-sized animal (for example, cattle). Even in animals of a single species, moreover, there arise differences depending on variety, individual and sex. In multiple pregnancy, differences in development are observed depending on the position of implantation in the uterine even in a litter.

On the other hand, a fertilized egg is made up of various cell organs and biological substances of each animal species. In the field of medical biology, there is a need to clarify how a difference in cell surface layer antigens, which participate in intercellular interaction, affects the development of a chimeric embryo. The size of a fertilized egg varies from species to species. For example, a mouse ovum is about 0.06 mm in size while a human ovum is three times as big as a mouse ovum. A fertilized egg of every mammal undergoes uniform division within the limited space in the clear gene. Therefore, the blastomere (egg cell) size is proportional to the ovum size, showing differences among species. A difference between cells likely results in a difference in three-dimensional structures in a chimera.

As will be shown in Examples hereinafter, chimeric embryoid bodies, even pulsating chimeric embryoid bodies, were formed from a small size mouse cells together with big size porcine cells. This fact indicates that chimeric animals can be constructed between distantly related species different from each other in embryological properties such as pregnancy period, cell surface layer antigen and size. Accordingly, it is suggested that one of the major difficulties in the construction of chimeric embryos between distantly related species might be thus overcome.

### ADVANTAGES OF THE INVENTION

There have been published a great number of science articles reporting that human cells are highly important as remedies, as vectors of genes to be used in gene therapy and as materials for constructing artificial organs or that human cells are absolutely short in supply, indicating the potential size of the market.

Because of with the problems of immune rejection and shortage of human cell sources, advances have been made in studies and tests on the method of treating cells, tissues or organs collected from patients and re-transplanting the same, utilization of cells collected from human wastes (placenta, umbilical cord, etc.) and a method of utilizing artificial aborted fetus cells. However, there still remains the problem of shortage in human cell supply, and it is particularly difficult to obtain cells of excellent qualities. As alternative methods, there have been developed methods of using porcine cells after same treatments (Porcine cardiomyocytes and their use in treatment of insufficient cardiac function: United States Patent 5,919,449; Isolated porcine pancreatic cells for use in treatment of disease characterized by insufficient insulin activity: United States Patent 5,961,972). However, these porcine cells are still inferior to human cells in stability *in vivo*, cell transfer efficiency and so on.

There are a considerable number of human cells which have been cultured and established *in vitro*. Human cells distributed by The Institute of Physical and Chemical Research (RIKEN) exclusively for laboratory purposes involve 200 or more established cell lines originating in more than 30 organs and tissues. However, these cells are not normal parenchymal cells and thus usable only in limited purposes. In particular, these cells are inadequate for transfer into the human body.

The most serious problem in using human cells on a commercial basis as a means of constantly supplying them resides in the ethics. From the ethical viewpoint, not only donors and recipients of human cells and doctors handling them but also animal welfare are taken into consideration.

### Examples

Now, a method of transferring foreign cells into mouse embryoid bodies and a method of developing a system of differentiation of the transferred foreign cells into cardiomyocytes will be described in detail by reference to the following Examples. However, the present invention is not construed as being restricted thereto.

### Example 1: Formation of cytotoxin gene expressed specifically in cardiomyocyte

### 1. Design of promoter (MLCpro)

A promoter region (MLCpro) specific to cardiomyocytes was designed in the following manner. First, genes expressed in the heart were examined using Internet Medline service. Based on titles and abstracts, documents including the term "cardiac specific" were screened. As a result, cardiac myosin light chain (MLC) and cardiac myosin heavy chain (MHC) were extracted. Since both of these genes have been clarified with respect to the structural genes and the upstream nucleotide sequences, it was considered that they were suitable for constructing recombinant DNA.

The position of the promoter region on DNA and its length vary from gene to gene. It is therefore preferred to employ a gene having a promoter region whose nucleotide sequence and position have been already clarified. When a gene is transferred into chromosome, the expression of the gene depends on the position on the chromosome (position-effect). Accordingly, it is desirable that a cloned gene is once returned into the animal individual for confirming its expression in cardiomyocytes. When embryoid bodies are employed as in this Example, it is desirable that the gene has been separated on the expression in the cardiomyocytes of embryoid bodies.

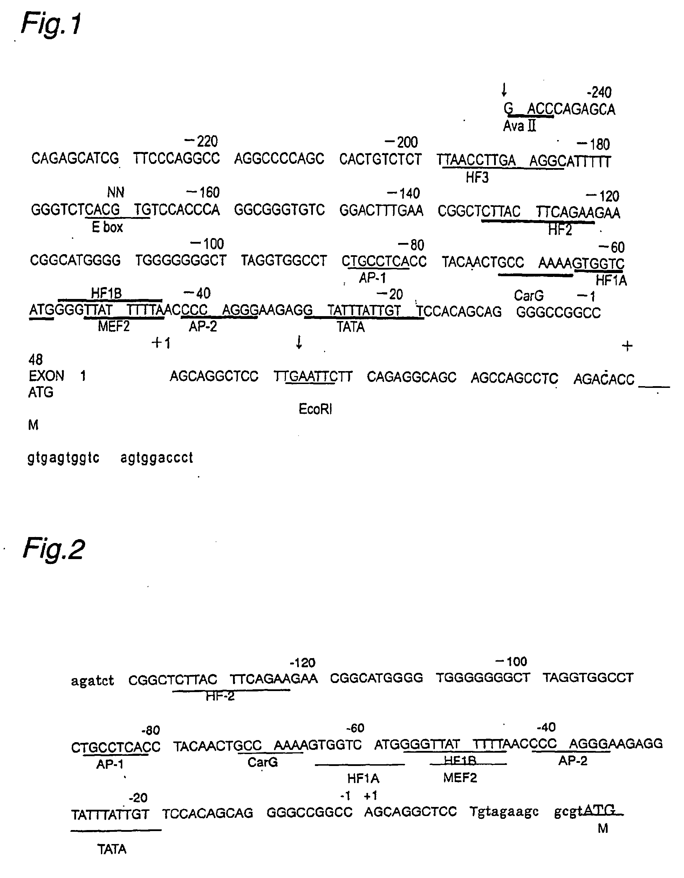

MLC2v (rat cardiac myosin light chain 2v) was chosen because it fulfils the above-described requirements (Fig. 1). Since it contained the region regulating cardiomyocyte-specific gene expression in a region of only 250 bp (base pairs), it was regarded as favorable by considering the purpose of experimentally obtaining MLCpro.

Different from early presumption, it was found that a long time and high cost would be required in a method of amplifying the 250 bp region by PCR with the use of rat genome as a template and, moreover, there arose a risk of failure. Thus, an attempt was made to synthesize MLCpro by a combination of the chemical synthesis of three template DNAs and PCR. As the results of a pre-experiment, it was clarified that MLCpro having a sequence comprising 11 G's with a T among them (Fig. 1: from -100 to -114) frequently suffered from misreading. Thus a DNA having such a length as allowing chemical synthesis as a whole was designed.

As the results of detailed re-examinations on documents concerning MLC2v, it appeared that HF3 (Fig. 1) on the discussion had been judged to be a unnecessary element in regulating cardiomyocyte-specific gene expression. It was also found that only the region containing TATA, AP-2, HF1A and HF1B could exhibit a sufficient function so long as 2 molecules of this region were ligated together.

Based on the consideration that it might be difficult to chemically synthesize a repeat of 2 molecules, a nucleotide sequence containing no HF3 and having such a length as allowing chemical synthesis as a whole was selected (Fig. 2).

### 2. Synthesis of MLCpro

The DNA shown in Fig. 2 was chemically synthesized with an automated DNA synthesizer (EXPEDITE) manufactured by Perkin Elmer. The thus synthesized MLCpro was integrated into a vector (pBluescript) marketed by Stratagene. Then the nucleotide sequence was analyzed by a DNA nucleotide sequence autoanalyzer (377 Sequencer) manufactured by Perkin Elmer, thereby confirming that the nucleotide sequence of MLCpro had been synthesized correctly.

### 3. Construction of MLCproTK, a gene expressing specifically in cardiomyocyte

Using as a template a vector (pKOselectTK) marketed by Takara Shuzo containing a suicidal gene (HSVTK: a gene of an enzyme converting a cytotoxin precursor into the cytotoxin) and the following 2 chemically synthesized DNAs as primers, the structural gene of HSVTK was amplified by PCR. TK-MET: TTTGGATCCTGTAGAAGCGCGTATG TK-PAA: TTTGCGGCCGCAGATCTGTAGTCAGGTTTAGTTCGTC (A set of primers for amplifying the cytotoxin precursor activating enzyme gene HSVTK.)

Using the following primers, the nucleotide sequence of the thus amplified DNA was confirmed with a DNA nucleotide sequence autoanalyzer. MLCTKF1: GTCTGCGTTCGACCAGGCTG (30S) MLCTKF2: AATGACAAGCGCCCAGATAA (360S) MLCTKF3: GCGAGCGGCTTGACCTGGCT (671S) MLCTKF3: TCCTGGATTACGACCAATCG (971S) MLCTKF4: TGAAGGCCCAGGGCTCGCAG (1337S) MLCTKR1: ACTTCCGTGGCTTCTTGCTG (100AS) (A set of primers for determining the nucleotide sequence of the amplified HSVTK gene.)

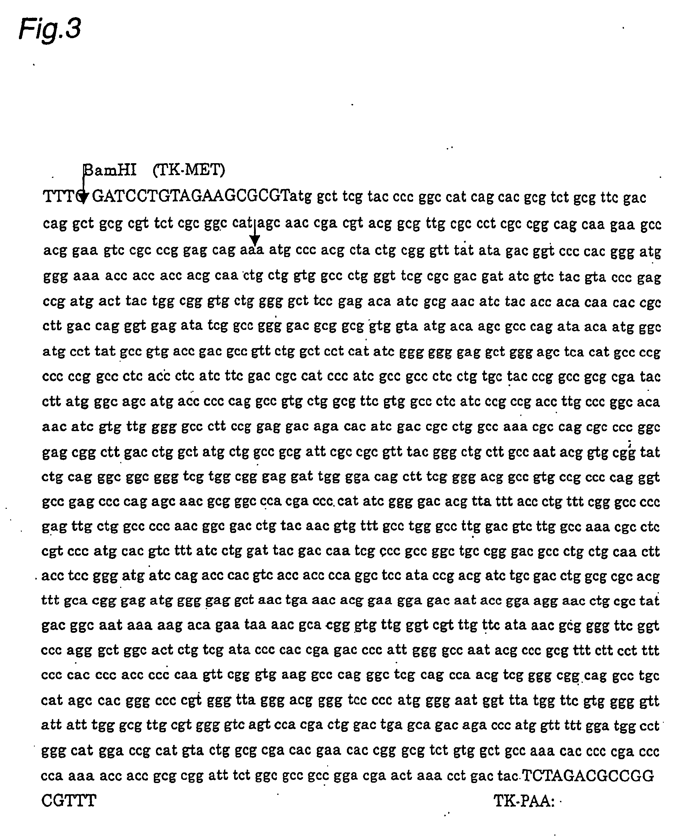

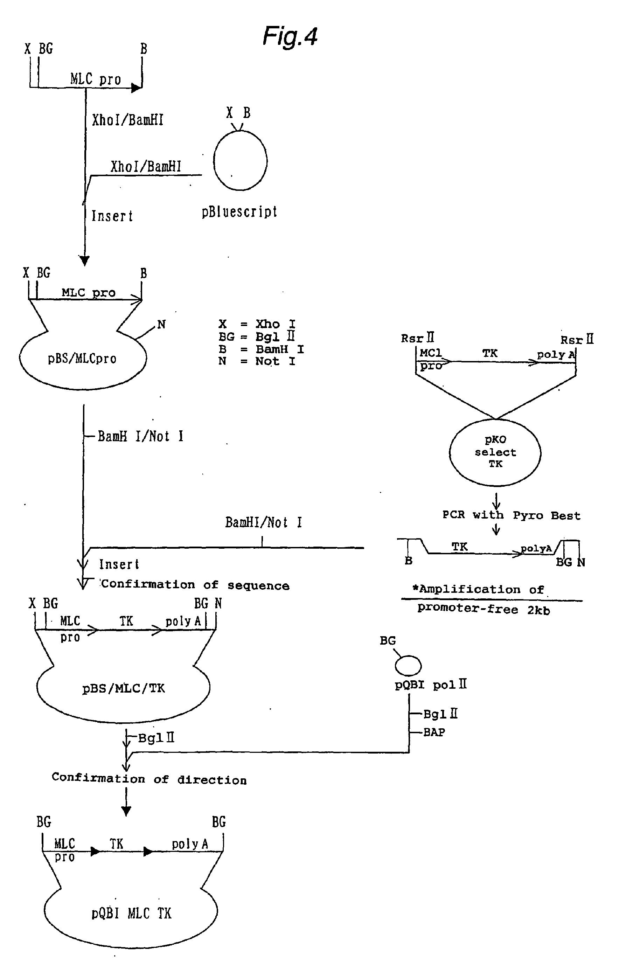

Fig. 3 shows the nucleotide sequence of the HSVTK structural gene obtained by the above procedure. This HSVTK gene was inserted into the 3'-side of MLCpro having been inserted into pBluescript (Fig. 4).

### 4. Construction of pQBIMLCTK an animal cell transfer vector (Fig. 4)

By the method as shown in Fig. 4, the above-described MLCproTK was inserted into the BglII site of pQBIpoI (marketed by Takara Shuzo), which was then introduced into *E. coli* JM109. Colonies showing ampicillin resistance were cultured, expanded and preserved as a stock. If necessary, a vector pQBIMLCTKDNA was prepared by a conventional method.

### Example 2: Construction of chimeric embryoid body

In this Example, production of a live-culture device with the use of porcine cells as the cells for organ and mouse ES cells as the cells for culture device will be illustrated.

As a general strategy, a more convenient co-culture method is first tested but if undesirable results are obtained, then the aggregation chimera method and the injection chimera method required a microscopic operation will become necessary. Since the co-culture method was successful in this example, the aggregation chimera method and the injection chimera method were omitted. The co-culture method employed herein was as follows.

### 1. Culture of ES cells

Mouse ES cells were purchased in a frozen state from Lifetec Oriental (Invitragen Japan) (Cat. No. YE9285300). After thawing, the ES cells were suspended in a medium, added into a 25 ml flask (FALCON 35-3014) and then cultured at 37°C in an atmosphere of 5% CO₂/95% air. As a feeder layer, use was made of STO cells which had been inactivated by treating with mitomycin C. The medium was replaced by a fresh one on a daily basis. When cells reached confluence, the cells were subcultured and frozen. The medium employed was prepared by mixing Buffalo rat liver cell conditioned medium into DMEM at a ratio of 3:2, which was supplemented with 0.1 mM of 2-mercaptoethanol, 0.1 mM of an MEM essential amino acid solution, a nucleoside solution (0.03 mM of adenosine, 0.03 mM of guanosine, 0.03 mM of cytidine, 0.03 mM of uridine and 0.03 mM of thymidine), 200 IU/ml of LIF, 10 ng/ml of bFGF and 20% of FCS. As a cell cryopreservation medium for the cells, use was made of Cell Banker (manufactured by Nippon Zenyaku Industries 905270).

### 2. Culture of GFP-expressing cells originating from porcine prolapsed blastocyst

A cell line established by Laboratory of Animal Reproduction, Tohoku University was employed (see the following document). The cells were cultured at 38.5°C in an atmosphere of 5% CO₂/95% air in a 100 ml culture dish (CORNING 100 mm/Tissue Culture Dish Cat. No. 25020), which had been treated with gelatin. After attaining confluence, the cells were subcultured and frozen. The medium was replaced by a fresh one once in every 2 days. The medium and the cell cryopreservation medium for the cells were the same as those employed above for the ES cells.

### 3. Construction of embryoid bodies

The ES cells and the GFP-expressing cells originating from porcine prolapsed blastocyst each attaining confluence were homogenized by treating with trypsin and plated on culture dishes (SUMILON 35 ml Dish Cat. NO. MS-1135) and mixed to give the cell counts as listed below. Then the cells were cultured at 37°C in an atmosphere of 5% CO₂/95% air.

**Table 1:**

| Ratio of TT2 ES cells to GFP-expressing porcine cells in each lot | | |
|---|---|---|
| Lot | Cell count (cells/dish) | |
| | TT2 ES cells | GFP-expressing porcine cells |
| 1 | 6 x 10⁵ | 0 |
| 2 | 5 x 10⁵ | 1 x 10⁵ |
| 3 | 4 x 10⁵ | 2 x 10⁵ |
| 4 | 3 x 10⁵ | 3 x 10⁵ |
| 5 | 2 x 10⁵ | 4 x 10⁵ |
| 6 | 1 x 10⁵ | 5 x 10⁵ |
| 7 | 0 | 6 x 10⁵ |

The day of the initiation of the culture was referred to as Day 0. On Day 3, the medium was replaced for the first time. Subsequently, the medium was replaced by a fresh one once in every 2 days. The medium employed was prepared by removing LIF from the medium used in the culture of the ES cells.

On Day 11, 20 embryoid bodies were transferred from each lot to another dish to provide a low-density culture group. Then the embryoid bodies were continuously cultured to observe the occurrence of pulsation and GFP expression in the low-density group and the high-density group (i.e., the lots remaining after transferring 20 embryoid bodies each).

The cells and the embryoid bodies were observed under an Olympus inversed system microscope (IMF-2). GFP was observed using an Olympus epi-illumination type fluorescent device (IMT2-RFL). To detect porcine cells, excitation light at 395 nm was employed.

### 4. Construction of TT2 having pQBI MLC TK introduced thereinto

ES cells were cultured in a 25 ml flask until confluence and then peeled by treating with trypsin and EDTA (TE treatment). Then a medium (DMEM+10%FCS) was added to inactivate trypsin and the cells were homogenized by pipetting.

To separate STO cells employed as the feeder cells from the ES cells, the cell suspension was fed into a 100 ml tissue culture dish having been treated with gelatin and cultured therein for 15 to 30 minutes. Then the STO cells thus adhered to the culture dish were removed.

The supernatant containing the ES cells was recovered and transferred into a centrifugal tube. Then the cells were homogenized again by pipetting. After counting the cells, centrifugation was performed at 1000 rpm for 5 minutes under cooling. After removing the supernatant, the cells were centrifugally washed with ice-cooled PBS(-) twice. The supernatant was removed and the cells were suspended in ice-cold PBS(-) to give a cell density of 4x10⁷/ml.

500 µl (2x10⁷ cells) of the above-described cell suspension and 20 µg of the pQBI MLC TK vector DNA constructed in Example 1 were transferred into a cuvette and allowed to stand on ice for 10 minutes. During this period, the cells and the vector DNA were mixed together by pipetting twice.

In electroporation, use was made of a BIO RAD gene pulsar. The cuvette was attached to a holder and pulse was applied (240 V-500 µF) once. Next, the cuvette was transferred onto ice and allowed to stand for 10 minutes.

After the completion of the electroporation, the cells were diluted with 50 ml of a medium and then transferred into a 100 ml tissue culture dish containing a layer of STO cells inactivated with mitomycin. Then the cells were cultured at 37°C in an atmosphere of 5% CO₂/95% air.

From the next day of the electroporation, cells having the gene introduced thereinto were selected using G418 (300 µg/ml) in the following manner. 3x10⁴ cells/dish of the ES cells were suspended in a medium containing G418 (GIBCO BRL Cat. NO. 11811) in 60 ml tissue culture dishes each containing a layer of STO cells (Neo-resistant). Then the ES cells were cultured at 37°C in an atmosphere of 5% CO₂/95% air. The medium employed was prepared by mixing Buffalo rat liver cell conditioned medium with DMEM at a ratio of 3:2, and then adding, to the resultant liquid mixture, 0.1 mM of 2-mercaptoethanol, 0.1 mM of an MEM essential amino acid solution, a nucleoside solution (0.03 mM of adenosine, 0.03 mM of guanosine, 0.03 mM of cytidine, 0.03 mM of uridine and 0.03 mM of thymidine), 200 IU/ml of LIF, 10 ng/ml of bFGF and 20% of FCS. During the first several days, the medium was replaced everyday. When the death of G418-sensitive cells became noticeable, the medium was replaced once in every 2 days. Culture was continued for about 8 days after the initiation of the screening.

### 5. Cloning of neomycin (Neo)-tolerant colonies

To ensure the homogeneity of the ES cells to be used in the experiment, it is necessary to isolate individual cell masses derived from a single cell. In this experiment, colonies were cloned by the penicillin cup method.

About 8 days after the initiation of the screening with the use of G418 (i.e., before the appearance of differentiated cells in colonies), the bottom face of each tissue culture was marked so as to surround individual colonies. After removing the medium, cells were washed with PBS(-). Then the PBS(-) was removed and a cloning cup (IWAKI GLASS Cat. NO. RING-12) coated with petrolatum at the bottom edge was placed on each marked colony. After adding TE into the cup, the cells were peeled off and transferred into a 24-well plate containing a layer of STO cells. Then culture was continued.

After reaching confluence, the cells were transferred into a 35 ml culture dish. After attaining confluence again, the cells were passaged into a 25 ml flask.

When the cells in the 25 ml flask reached confluence, passage was carried out again. After reaching confluence, the cells were suspended in Cell Banker (manufactured by Nippon Zenyaku Industries 905270) to give 5x10⁶ cells per tube (1 ml) and stored in a frozen state at -80°C.

### 6. Construction of chimeric embryoids between TT2(pQBIMLCTK) cells and porcine cells

The above procedure of "Construction of embryoid bodies" was followed but adding 5 µg/ml of ganciclovir (Wako Pure Chemical Industries, 074-04483) to the medium.

### Results and Discussion

After sowing the cells at the ratios as given in Table 1 in the culture dishes and culturing for 11 days, the number of formed embryoid bodies in each lot were counted. The results are as follows: 176 in Lot 1; 202 in Lot 2; 162 in Lot 3; 264 in Lot 4; 192 in Lot 5; 168 in Lot 6; and 0 in Lot 7. Thus, it was clarified that no embryoid body was formed by using the porcine cells alone. On Day 11 of the culture, pulsating embryoid bodies were observed for the first time (0 in Lots 1 to 3; 1 in Lot 4; 2 in Lot 5; and 7 in Lot 6).

**Table 2:**

| Pulsation in embryoid bodies cultured till Day 16 | | | |
|---|---|---|---|
| Lot | High-density lot | Low-density lot | |
| | Day 16 | Day 13 | Day 16 |
| 1 | 2/156 (1%) | 0/20 (0%) | 4/20 (20%) |
| 2 | 17/182 (9%) | 4/20 (20%) | 5/20 (25%) |
| 3 | 19/142 (13%) | 2/20 (10%) | 5/20 (25%) |
| 4 | 23/244 (9%) | 5/20 (25%) | 7/20 (35%) |
| 5 | 23/172 (13%) | 5/20 (25%) | 5/20 (25%) |
| 6 | 37/148 (25%) | 7/20 (35%) | 6/20 (30%) |

A comparison of the data on Day 16 listed in Table 2 indicates that pulsating embryoid bodies appeared at higher ratios in case of restricting the number of the embryoid bodies. It was also observed that pulsation started earlier in embryoid bodies in the lot with a high ratio of the porcine cells (Lot 6). In this case, however, only a small number of GFP-expressing cells were incorporated in each embryoid body regardless of the occurrence of pulsation and fluorescence was observed in only part of luminescent embryoid bodies. In the lot with relatively low ratios of the porcine cells (Lots 2, 3 and 4), on the contrary, pulsation started more slowly. With the passage of time, however, the numbers of pulsating embryoid bodies in these lots attained to a level comparable to that in the lot with a high porcine cell ratio (Table 3). Moreover, stronger fluorescence over a broader scope was observed in the lots with lower porcine cell ratios.

**Table 3:**

| Pulsation and GFP expression in embryoid bodies cultured at low density | | | | | | |
|---|---|---|---|---|---|---|
| Lot | Day 21 | | | Day 40 | | |
| | Pulsation | Fluorescence | Pulsation & fluorescence | Pulsation | Fluorescence | Pulsation & fluorescence |
| 1 | 4(20%) | -(-) | -(-) | 1( 5%) | -(-) | -(-) |
| 2 | 4(20%) | 7(35%) | 2(10%) | 3(15%) | 0( 0%) | 0( 0%) |
| 3 | 2(10%) | 6(30%) | 0( 0%) | 1( 5%) | 2(10%) | 0( 0%) |
| 4 | 6(30%) | 4(20%) | 2(10%) | 0( 0%) | 4(20%) | 0( 0%) |
| 5 | 4(20%) | 6(30%) | 2(10%) | 0( 0%) | 0( 0%) | 0( 0%) |
| 6 | 4(20%) | 10(50%) | 2(10%) | 2(10%) | 3(15%) | 0( 0%) |

Pulsation: number of pulsating embryoid bodies. Fluorescence: number of embryoid bodies having GFP-expressing cells
incorporated therein.
Pulsation & fluorescence: number of embryoid bodies showing
pulsation and having GFP-expression cells
incorporated therein.

Table 3 indicates that the number of pulsating embryoid bodies and the number of embryoid bodies having GFP-expression cells incorporated therein decreased with the passage of time. However, relatively strong pulsation was observed in most of the embryoid bodies showing pulsation on Day 40 and pulsation at almost the same strength was continuously observed thereafter.

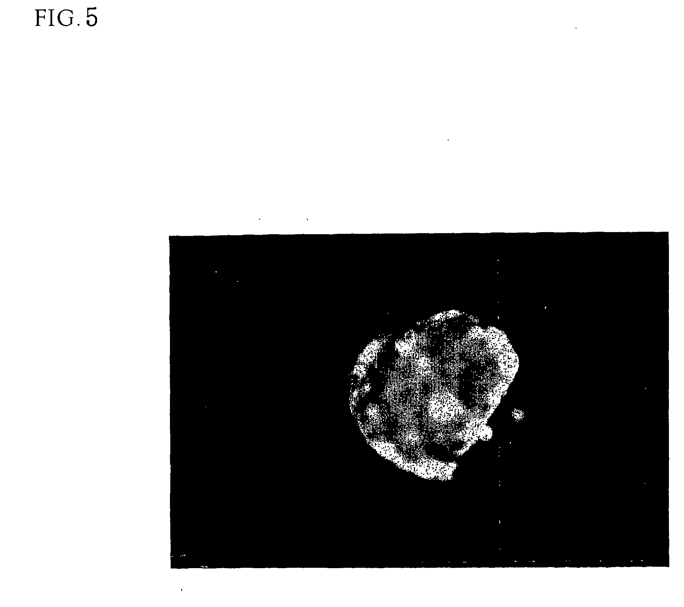

Fig. 5 shows a chimeric embryoid body prepared in the presence of ganciclovir and subjected to the removal of the cardiomyocyte having pQBIMLCTK transferred thereinto. This embryoid body shows pulsating cells with green fluorescence (i.e., porcine cells).

### Example 3: Development of mouse chimeric embryo having porcine embryo-origin cells transferred thereinto

### 1. Gene transfer into mouse embryo

A mouse cardiomyocyte-specifically expressing a cytotoxin (HSVtk) was constructed by transferring MLCproTK gene in accordance with a common method of constructing transgenic mice (Hogan B et al. (1986) Cold Spring Harbor).

### • Preparation of DNA solution

DNA of a plasmid pBS/MLC/TK containing MLCproTK was cleaved with a restriction enzyme BglII and the MLCproTK fragment was isolated by agarose electrophoresis. This DNA was dissolved in PBS to give a concentration of 5 µg/ml and then centrifuged at 15000 rpm for 30 minutes to thereby remove insoluble matters.

### • Collection of fertilized eggs in pronucleus stage

Super-ovulated female B6C3F1 mice were fed together with male mice of the same strain. On the next morning, fertilized eggs in the pronucleus stage were collected from ampulla of the uterine tube of females having been confirmed as mated by using vaginal plugs. The fertilized eggs in the pronucleus stage thus collected were immersed in an M16 solution containing 1 mg/ml of hyaluronidase and oosphere cells were removed therefrom by lightly pipetting.

### • Injection of DNA solution into mouse fertilized eggs

The following procedures were carried out under an inverted differential interference microscope (manufactured by Nikon) provided with a micromanipulator (manufactured by Narishige) and a microinjector (manufactured by Narishige). The fertilized eggs obtained above were fixed by sucking under negative pressure of a holding pipette and 1 to 2 pl of the above-described DNA solution was injected into the male pronucleus from an injection pipette. After culturing till the next day, mouse fertilized eggs having developed into the bicellular stage were selected. Then 15 to 30 eggs per animal were transplanted into the uterine tube of CD-1 females under false pregnancy. Three weeks after the birth, DNAs collected from the tail of infants immediately after weaning were calibrated by the PCR method to thereby give transgenic mice having MLCpro transferred thereinto.

Three strains of the male transgenic mice of the first generation were selected and a strain in which the transferred MLCproTK functioned was screened by the following method. Namely, these male transgenic mice were mated with super-ovulated normal female mice. To females having been confirmed as mated by using vaginal plugs, 50 mg/kg of ganciclovir was intravenously injected twice a day from Day 4 after the mating. On Day 12 after the mating, these animals were subjected to abdominal section so as to examine abnormality in fetal development and implantation traces. Thus, partners (MLCproTK mice) of females showing no surviving fetus were screened.

### • Transfer of porcine cells into transgenic mouse embryos by injection

Super-ovulated female B6C3F1 mice were mated with the above-described male MLCproTK mice and fertilized eggs in the blastocyst stage carrying the MLCproTK gene were collected. By operating a microinjector under a microscope, about 20 porcine cells having the GFP gene transferred thereinto were sucked at the tip of an injection pipette. Then the tip of the injection pipette was inserted into the blastocoel of the above-described mouse fertilized eggs in the blastocyst stage fixed on a holding pipette and about 15 porcine embryo-origin cells in the pipette were injected thereinto by operating the microinjector.

25 to 30 fertilized eggs, which had the porcine cells injected thereinto as described above, were transplanted into the uterine of female CD-1 mice under false pregnancy within several hours. From Day 2 after the transplantation, 50 mg/kg of ganciclovir was intravenously injected into the hosts twice a day. On Day 10 after the transplantation, these animals were subjected to abdominal section. The fetuses were taken out and observed under a fluorescent microscope. Thus, accumulation of fluorescent porcine cells was observed in the heart.

Referential Patents
(1) US Patent 5,888,816 Mar. 30, 1999
   "Cell culbures of and cell culturing method for nontransformed pancreatic thyroid, and paranthyroid cells"
   Assignee: Human Cell Culture, Inc. (East Sebago, ME)
(2) US Patent 5,919,449 Jul. 6, 1999
   "Porcine cardiomyocytes and their use in treatment of insufficient cardiac function"
   Assignee: Diacrin, Inc. (Charleston, MA)
(3) US Patent 5,961,972 Oct. 5, 1999
   "Isolated porcine pancreatic cells for use in treatment
   of diseases characterized by insufficient insulin activity"
   Assignee: Diacrin, Inc. (Charleston, MA)
(4) US Patent 5, 679,340 Oct. 21, 1997
   "Cells with multiple altered epitopes on a surface antigen for use in transplantation"
   Assignee: Diacrin, Inc. (Charleston, MA)
(5) US Patent 5,705,732 Jan. 6, 1998
   "Universal donor cells"
   Assignee: Oklahoma Medical Research, Foundation
   (Oklahoma City, OK)

### Referential Document on MLCpro

(1) Scott A, Henderson, et al.
   Structure, Organization, and Expression of the Rat Cardiac Myosin Light Chain-2-Gene: IDENTIFICATION OF A 250-BASE PAIR FRAGMENT WHICH CONFERS CARDIAC-SPECIFIC
   EXPRESSION
   THE JOURNAL OF BIOLOGICAL CHEMISTRY 264, 18142-18148, 1989
(2) HONG ZHU, et al.
   A Conserved 28-Base-Pair Element (HF-1) in the Rat Cardiac Myosin Light-Chain-2 Gene Confers Cardiac-Specific and α-Adrenergic-Inducible Expression in Cultured Neonatal Rat Myocardial Cells
   MOLECULAR AND CELLULAR BIOLOGY 11, 2273-2281 (1991)
(3) Robert S. Ross, et al.
   An HF-1a/HF-1b/MEF-2 combinatorial element confers cardiac ventricular specificity and establishes an anterior-posterior gradient of expression,
   DEVELOPMENT 122, 12799-1809 (1996)
   Documents Reporting Establishment of Porcine Cell Line

(1) Establishment of ES-like cells from porcine blastocysts
   constructed *in vitro*
   Kazuchika Miyoshi, Hideaki Sato
   Nippon Jusei Chakusho Gakkai Zasshi 15, 180-182 (1998)
(2) Effect of hyaluronic acid on the development of porcinel-
   cell embryos produced by a conventional or new *in vitro*
   maturation/fertilization system
   K. Miyoshi, M, Umezu, E. Sato
   Theriogenology 51, 777-784 (1999)
(3) Establishment of a porcine cell line from *in vitro*-produced blastocyst and transfer of cells into enucleated oocytes
   K. Miyoshi, Y. Taguchi, Y. Sendai, H. Hoshi, E. Sato Biology of Reproduction (under submission)

## Claims

1. A method of producing normal parenchymal cells, tissues or organs (hereinafter referred to as "organ or the like") originating in human or nonhuman mammal which comprises the following steps:
(1) preparing cells which are obtained from a mammal of the same species as the target organ or the like to be produced and capable of differentiating at least into the target organ or the like (hereinafter referred to as "cells for organ");
(2) preparing cells from a nonhuman mammal of a species different from the species of the target organ or the like to be produced and have pluripotency or totipotency (hereinafter referred to as "cells for live-culture device");
(3) introducing a DNA fragment containing a promoter region functioning specifically in the target organ or the like to be produced and a structural gene encoding an active cytotoxin or an enzyme to activate a cytotoxin precursor which is ligated under the control of said promoter region into the genome of the cells for live-culture device as described in (2);
(4) culturing the cells for organ as described in (1) together with the cells for culture device as described in (3) to form a chimeric cell mass;
(5) culturing the chimeric cell mass under conditions adequate for the development thereof to provide a live-culture device made of a mammal mainly having the characters originating in the cells for live-culture device, or its embryo or embryoid body, while specifically expressing the active cytotoxin or the cytotoxin precursor activating enzyme in the cells originating in the cells for culture device of the target organ or the like formed in the course of the development of the live-culture device and, in the case of expressing the cytotoxin precursor activating enzyme, adding the cytotoxin precursor; and
(6) allowing the live-culture device to produce the target organ or the like which is made up of the cells originating in the cells for organ and substantially free from cells originating in the cells for culture device.

2. A method according to claim 1 wherein said cytotoxin precursor is ganciclovir, said enzyme converting ganciclovir into a cytotoxin is HSVtk (herpes symplex virus thymidine kinase), and said promoter region is the promoter of rat myosin light chain 2v gene which functions exclusively in cardiomyocytes.

3. A method according to claim 1 or 2 which aims at producing cardiomyocytes or a heart originating in a human or a nonhuman mammal.

4. A method according to claim 1 or 3 for producing an organ selected from the group consisting of organs made up of cells characteristic of said organ.

5. A method according to any of claims 1 to 4 wherein said cells for live-culture device are selected from the group consisting of cells of mammals usable in embryo manipulations such as pig, chimpanzee, gorilla, orangutan, monkey, dog, cattle, horse, sheep, goat, rat and mouse.

6. A recombinant DNA to be introduced into the genome of the cells for culture device in the method according to claim 1, comprising a promoter region functioning specifically in the target organ or the like and a gene encoding an enzyme for converting a cytotoxin precursor into the cytotoxin, wherein the expression of the enzyme gene is under the control of said promoter region.

7. A recombinant DNA according to claim 6 wherein said cytotoxin precursor is ganciclovir, said enzyme for converting ganciclovir into a cytotoxin is HSVtk (herpes symplex virus thymidine kinase), and said promoter region is the promoter of rat myosin light chain 2v gene which functions exclusively in cardiomyocytes.

8. A nonhuman mammal having normal parenchymal cells of a foreign mammal, which is either a human or nonhuman mammal, a tissue made up of normal cells of the foreign mammal and secretion products thereof, and/or an organ of the foreign mammal.

9. A live-culture device wherein a culture device for producing normal parenchymal cells, a tissue and/or an organ of a human or nonhuman mammal undergoes morphological and biochemical changes under a definite law to thereby support the proliferation of target cells and, at the same time, continuously and three-dimensionally induce the differentiation and maturation of the cells, thereby forming the tissue or organ.

10. A live-culture device according to claim 9 which is an embryo of a nonhuman mammal carrying a recombinant DNA according to claim 6 or 7 introduced thereinto.

11. A live-culture device according to claim 9 which comprises cells (for example, embryonic stem cells) carrying a recombinant DNA according to claim 6 or 7 introduced thereinto, wherein the cells can form a construct (for example, an embryoid body) which is necessarily and sufficiently capable of supporting the proliferation, differentiation and maturation of the target normal parenchymal cells and undergoes changes under a definite law.

12. A method of constructing a chimeric embryo or a chimeric embryoid body between a non-human mammalian embryo or cells of a mammal including a human which are capable of forming an embryoid body and undifferentiated cells of a foreign nonhuman mammal.

13. Cells and a cell-like construct wherein
undifferentiated cells of a foreign animal such as human being or a nonhuman mammal are cells capable of differentiating into target cells, tissue or organ which are selected from the group consisting of female/male germ cells and precursor cells thereof, embryonic cells, embryo-derived pluripotent cells, embryonic stem cells, embryonic stem cell-derived pluripotent cells, stem cells of various tissues and dedifferntiated somatic cells, and whereby these cells can be developed into a structure having the whole functions or a part of the functions of the target cell, tissue or organ, for example, artificially treated cells such as genetically modified cells, modified cells by artificial treatment of the part of the cell functions, cells partly substituted by an artificial substance and artificially synthesized cells.

14. A method of promoting the
proliferation/differentiation of foreign mammalian cells (for example, human cells) in a live-culture device into target cells or a tissue or organ while removing specific cells, which comprises adding a cytotoxin precursor into the live-culture device and exclusively removing the cells in which a gene of an enzyme capable of converting the cytotoxin precursor into the cytotoxin is expressed.
